# FASCICULE DE BREVET EUROPEEN

(11) **EP 0 326 471 B1**
(45) Date de publication et mention de la délivrance du brevet: **14.09.1994**
(21) Numéro de dépôt: 89400178.3
(22) Date de dépôt: 23.01.1989
(51) Int. Cl.: H05G 1/60, G01N 23/04, A61B 6/03

(54) **Appareil de tomographie à rayons X**
Vorrichtung für Röntgenstrahlentomographie
Apparatus for X-ray tomography

(30) Priorité: 25.01.1988 FR 8800784
(43) Date de publication de la demande: 02.08.1989
(73) Titulaire: COMMISSARIAT A L'ENERGIE ATOMIQUE, 75015 Paris Cédex 15 (FR)
(72) Inventeur: Cuzin, Marc, F-38700 La Tronche (FR); Glasser, Francis, F-38560 Jarrie (FR)
(74) Mandataire: Signore, Robert

(56) Documents cités:
- EP-A- 0 213 213
- DE-A- 3 244 636
- FR-A- 2 260 252
- FR-A- 2 365 328
- GB-A- 2 004 436
- US-A- 4 070 707
- US-A- 4 366 382
- US-A- 4 484 340

## Description

La présente invention concerne un appareil de tomographie utilisant une source pulsée de rayons X.

Cette invention s'applique notamment au contrôle industriel d'objets fortement absorbants et de grandes dimensions, pour lesquels on souhaite reconstruire des images de coupes planes, en utilisant une source pulsée de rayonnement X de haute énergie (par exemple supérieure à quelques 100 KeV), et des moyens de traitements de signaux issus de détecteurs de rayonnements ayant traversé cet objet. Cette invention peut aussi s'appliquer au contrôle d'objets peu absorbants et de plus faibles dimensions.

On sait qu'un type d'appareil de tomographie à rayons X qui permet d'obtenir l'image d'au moins une coupe plane d'un objet, comprend une source de rayons X fournissant des impulsions de haute énergie, à une fréquence prédéterminée. Cet appareil comprend aussi un collimateur primaire pour que les impulsions traversent l'objet dans un plan de coupe et au voisinage de celui-ci. Des moyens sont généralement prévus pour supporter l'objet et pour le déplacer en translation et/ou en rotation. Ce type d'appareil comprend aussi une barrette de détecteurs de mesure, recevant les impulsions d'énergie atténuée ayant traversé l'objet et un collimateur secondaire placé entre l'objet et la barrette de détecteurs. Ces détecteurs fournissent respectivement sur des sorties, des impulsions électriques de détection, représentatives des impulsions reçues d'énergie atténuée, provenant de l'objet. Enfin, cet appareil de type connu comprend des moyens de traitements et de visualisation, reliés aux sorties des détecteurs pour fournir l'image de chaque coupe de l'objet. Les signaux issus des détecteurs sont généralement codés et numérisés pour permettre leur traitement par un calculateur. Dans ce type d'appareil, les détecteurs peuvent être constitués par des photomultiplicateurs ou des photodiodes associés respectivement a un scintillateur.

Dans ce type de dispositif connu, la source pulsée de rayons X ne présente pas une stabilité parfaite et les moyens de détection peuvent présenter des dérives en température et des courants d'obscurité et dans le cas d'un objet radioactif il existe un signal de détection parasite dont il n'est pas tenu compte pour le traitement des signaux. Il en résulte que l'image de chaque coupe obtenue n'est pas parfaite. C'est le cas par exemple du dispositif décrit dans le document DE-A-3244 636.

Lorsque les moyens de détection utilisés sont constitués par des photomultiplicateurs ou des photodiodes associés à des scintillateurs, ces scintillateurs vieillissent sous l'effet du rayonnement de sorte que leur transparence, et donc leur rendement, se trouvent altérés après absorption d'une certaine dose de rayons X. Il est alors nécessaire de régénérer les scintillateurs par un cycle thermique.

Il en résulte que tout appareil de tomographie qui utilise des scintillateurs, est souvent indisponible. De plus, le volume occupé par un ensemble de scintillateurs et de photodétecteurs (photomultiplicateurs ou photodiodes) limite les possibilités de réaliser des moyens de détection a faible pas géométrique, ce qui limite fortement le nombre de points de mesures. Enfin, la dynamique Limitée des photomultiplicateurs implique l'utilisation de photodiodes pour les objets à forte dynamique d'absorption, mais dans ce cas il est nécessaire de prendre en compte deux rendements de conversion énergétique : le rendement relatif à la conversion des photons X en photons visibles et le rendement relatif à la conversion des photons visibles en charges électriques. Il en résulte une perte de sensibilité très importante des appareils ainsi constitués et l'introduction d'un bruit statistique très important. Le rendement de scintillation est en général très faible (inférieur a 10%).

De manière générale, on préfère donc utiliser des moyens de détection qui permettent d'effectuer une conversion directe des photons X en charges électriques. Ces moyens de détection sont généralement constitues par des barrettes de détecteurs à semi-conducteur.

Ces détecteurs sont utilisés de façon connue avec des sources de rayonnement continu de faible énergie (typiquement avec des générateurs de rayons X alimentés par une tension continue de l'ordre de quelques 10 k volt). Ces détecteurs sont répartis sous forme de barrette (ou de mosaïque), chaque détecteur étant constitué par une pastille semi-conductrice de faibles dimensions (de l'ordre du mm) intercalée entre deux électrodes, le rayonnement à détecter arrivant sous incidence normale aux plans des détecteurs et des électrodes.

Cette géométrie des détecteurs ne permet pas de les utiliser avec un bon rendement, pour des hautes énergies. En effet, avec une telle structure, l'augmentation de l'épaisseur de la pastille semi-conductrice, rendue nécessaire pour obtenir une bonne efficacité en haute énergie, introduit une distance de collection des charges créées important. Ceci introduit deux défauts majeurs :
- les charges peuvent se recombiner avant d'atteindre une électrode,
- le temps de collection maximum des charges est important.

Les appareils de tomographie connus fonctionnant avec des sources de rayons X de haute énergie n'utilisent pas de ce fait des détecteurs à semi-conducteur.

Dans les appareils de tomographie connus à source pulsée, les signaux fournis respectivement par les détecteurs sont des signaux impulsionnels qui présentent une traînée très importante lorsque le flux de rayonnements X reçus a une énergie très importante. Il en résulte que le signal impulsionnel fourni par un détecteur après réception d'une impulsion de rayons X ayant traversé l'objet, peut se superposer au signal impulsionnel fourni par ce détecteur lors de la réception de l'impulsion de rayons X reçue précédemment. De plus, ce détecteurs présentent au cours de leur irradiation par les impulsions provenant de l'objet, une dérive en température assez importante.

Ces appareils de tomographie connus fonctionnant avec des sources de rayons X de haute énergie traitent les signaux fournis par les détecteurs, sans tenir compte de la traînée du signal électrique impulsionnel fourni par chaque détecteur, des dérives en température provoquées par l'irradiation de ces détecteurs, du courant d'obscurité de chaque détecteur. Ces appareils ne tiennent pas compte non plus de l'instabilité de la source pulsée de rayons X et du rayonnement éventuel émis par l'objet lui-même (objet radioactif). Il en résulte que l'image de chaque coupe de l'objet n'est pas de bonne qualité.

L'invention a pour but de remédier à ces inconvénients en réalisant un appareil de tomographie à rayons X utilisant une source pulsée de haute énergie et au moins une barrette de photodétecteurs, cet appareil permettant, grâce à des moyens appropriés, de traiter le signal électrique impulsionnel issu de chaque détecteur, en tenant compte de la traînée de ce signal, de la dérive des détecteurs en température, du courant d'obscurité, de l'instabilité de la source et du rayonnement éventuel émis par l'objet. Les détecteurs de l'appareil de l'invention sont de façon avantageuse de type à semi-conducteur, conçus pour avoir un bon rendement en hautes énergies et pour éventuellement éviter toute diffusion de rayonnements d'un détecteur vers un autre détecteur, au cours du fonctionnement.

L'invention a pour objet un appareil de tomographie à rayons X, pour obtenir l'image d'au moins une coupe plane d'un objet, comprenant une source de rayons X fournissant des impulsions de haute énergie, à une fréquence prédéterminée, un collimateur primaire pour que ces impulsions traversent l'objet au voisinage d'un plan de coupe, au moins une barrette de détecteurs de mesure recevant les impulsions d'énergie atténuée ayant traversé l'objet et un collimateur secondaire, ces détecteurs fournissant respectivement sur des sorties des impulsions électriques de détection, représentatives des impulsions d'énergie atténuée provenant de l'objet, des moyens de traitement et de visualisation reliés aux sorties des détecteurs de mesure pour fournir l'image de ladite coupe et des moyens capables de déplacer en translation et/ou en rotation l'objet et/ou l'ensemble source, collimateurs primaire et secondaire, barrette de détecteurs, les moyens de traitement et de visualisation comprenant un ensemble de circuits amplificateurs-intégrateurs ayant des entrées respectivement reliées aux sorties des détecteurs de mesure, chaque amplificateur-intégrateur ayant une entrée pour commander la durée d'intégration, un calculateur relié à une mémoire et à des moyens de visualisation et ayant des sorties de commande respectivement reliées aux entrées de commande des circuits intégrateurs-amplificateurs, la mémoire du calculateur contenant un programme de séquencement et de traitement pour commander chaque circuit intégrateur-amplificateur pour que chaque impulsion de détection fournie par un détecteur correspondant, soit intégrée sur une première durée dite de mesure brute, et pour intégrer cette impulsion de détection sur au moins une deuxième durée dite de correction, dans l'intervalle de temps qui sépare cette impulsion de détection de l'impulsion de détection suivante, ce circuit intégrateur-amplificateur fournissant sur une sortie une première impulsion de mesure brute et une deuxième impulsion de correction ayant respectivement les première et deuxième durées, l'amplitude de la première impulsion étant représentative de l'énergie de l'impulsion atténuée provenant de l'objet et reçue par le détecteur, l'amplitude de la deuxième impulsion étant représentative de la traînée de l'impulsion électrique de détection fournie par le détecteur et du rayonnement éventuel de l'objet et du courant de fuite du détecteur, les circuits amplificateurs-intégrateurs étant regroupés en au moins un ensemble, les circuits de chaque ensemble ayant une sortie commune reliée à un convertisseur analogique-numérique ayant une sortie reliée au calculateur pour fournir des valeurs numériques de mesures brute et de correction, respectivement représentatives des amplitudes des première et deuxième impulsions de mesure et de correction, le calculateur traitant ces valeurs numériques pour chaque impulsion de détection pour fournir une valeur numérique effective de mesure de l'énergie de l'impulsion atténuée reçue par le détecteur, cette valeur numérique effective étant enregistrée dans la mémoire, les valeurs numériques effectives ainsi enregistrées pour tous les détecteurs étant utilisées par le calculateur pour commander l'affichage de l'image de ladite coupe par les moyens de visualisation.

Selon un premier mode de réalisation de l'invention, l'appareil comprend une barrette de détecteurs de compensation, identique à la barrette de détecteurs de mesure et située à proximité de cette barrette de détecteurs de mesure, cette barrette de détecteurs de compensation comportant des moyens pour la rendre opaque aux impulsions d'énergie atténuées provenant de l'objet, chaque circuit amplificateur-intégrateur comportant un amplificateur différentiateur ayant une première entrée reliée au détecteur de mesure correspondant, et une deuxième entrée reliée au détecteur de compensation correspondant, chaque amplificateur différentiateur fournissant sur une sortie une impulsion de détection amplifiée et compensée en fonction de la dérive en température et du courant d'obscurité du détecteur de mesure correspondant, la sortie de cet amplificateur différentiateur étant reliée à une entrée d'un intégrateur ayant l'une desdites entrées de commande de la durée d'intégration et fournissant sur une sortie lesdites impulsions de mesure et de correction chaque détecteur comportant une pastille semi-conductrice, chaque pastille ayant la forme d'un parallélépipède allongé défini par sa largeur, sa hauteur et sa profondeur, une face de ce parallélépipède définie par la largeur et la hauteur étant située en regard de l'objet, deux autres faces parallèles de ce parallélépipède définies par la hauteur et la profondeur portant respectivement deux électrodes d'alimentation électrique, l'une de ces électrodes fournissant lesdites impulsions de détection. De façon avantageuse, chaque électrode est recouverte d'une couche de blindage opaque aux rayons reçus par le détecteur et évitant la diffusion dans chaque détecteur voisin.

Selon une mode particulier de réalisation, chaque circuit amplificateur-intégrateur comprend un montage intégrateur à amplificateur opérationnel, une entrée de cet amplificateur opérationnel recevant l'impulsion de détection provenant du détecteur correspondant, une entrée de commande de la durée d'intégration du montage intégrateur étant reliée à une sortie de commande correspondante du calculateur, une sortie de cet amplificateur opérationnel étant reliée à une borne d'un interrupteur ayant une entrée de commande d'ouverture ou de fermeture reliée à une sortie de commande correspondante du calculateur, une autre borne de cet interrupteur étant reliée à l'entrée du convertisseur analogique-numérique correspondant.

Selon un autre mode particulier de réalisation, chaque amplificateur intégrateur comprend un montage intégrateur à amplificateur opérationnel, une entrée de cet amplificateur opérationnel recevant l'impulsion amplifiée de détection provenant de l'amplificateur différentiateur correspondant, une entrée de commande de la durée d'intégration du montage intégrateur étant reliée à une sortie de commande correspondante du calculateur, une sortie du montage à amplificateur opérationnel étant reliée à une borne d'un interrupteur ayant une entrée de commande d'ouverture ou de fermeture reliée à une sortie de commande correspondante du calculateur, une autre borne de cet interrupteur étant reliée à entrée du convertisseur analogique-numérique correspondant.

Selon un autre mode de réalisation, l'appareil comporte des moyens numériques d'indication de température situés à proximité de la barrette de détecteurs de mesure, une sortie de ces moyens d'indication fournissant un signal numérique d'indication de la valeur de la température à proximité de la barrette de détecteurs de mesure, cette sortie étant reliée à une entrée du calculateur pour que celui-ci effectue un traitement de correction des valeurs numériques de mesures effectives enregistrées, pour corriger ces valeurs numériques selon la dérive en température des détecteurs de mesure.

Selon une autre caractéristique, l'appareil comporte en outre un détecteur de référence recevant les impulsions de la source et fournissant sur une sortie des impulsions de détection de référence représentatives de l'énergie de la source, un convertisseur analogique-numérique ayant une entrée reliée à la sortie du détecteur de référence et fournissant sur une sortie, une valeur numérique représentative de l'énergie de la source, cette sortie étant reliée à une entrée du calculateur pour que celui-ci effectue un traitement de correction des valeurs numériques effectives enregistrées, pour corriger ces valeurs selon les variations d'énergie de la source.

Selon une autre caractéristique, une couche électriquement isolante est intercalée entre chaque électrode et la couche de blindage.

Les caractéristiques et avantages de l'invention ressortiront mieux de la description qui va suivre donnée en référence aux dessins annexés dans lesquels :
- la figure 1 représente schématiquement un appareil de tomographie à rayons X conforme à l'invention,
- la figure 2 est un diagramme représentant les signaux impulsionnels obtenus à la sortie d'un détecteur de l'appareil de l'invention,
- la figure 3 représente schématiquement un premier mode de réalisation de barrettes de détecteurs de mesure et de compensation utilisés dans l'appareil de l'invention,
- la figure 4 représente schématiquement un autre mode de réalisation d'un circuit amplificateur-intégrateur utilisé dans l'appareil de l'invention,
- la figure 5 représente schématiquement un autre mode de réalisation d'un amplificateur-intégrateur utilisé dans l'appareil de l'invention,
- la figure 6 représente schématiquement un premier mode de réalisation d'une barrette de détecteurs de mesures utilisés dans l'appareil de l'invention,
- la figure 7 représente schématiquement un autre mode de réalisation d'un barrette de détecteurs de mesure utilisés dans l'appareil de l'invention.

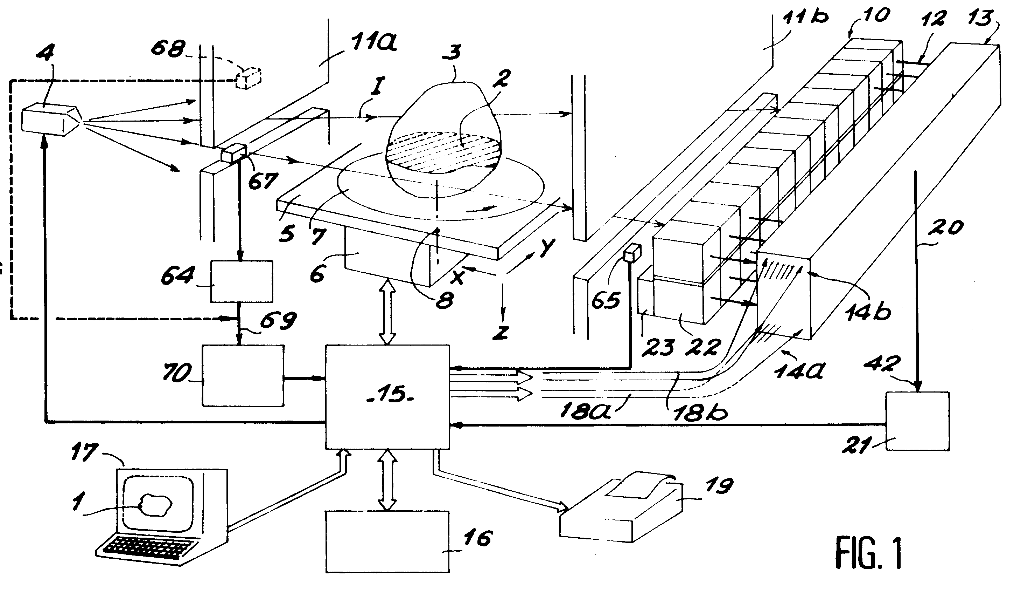

L'appareil de tomographie à rayons X représenté schématiquement sur la figure 1 permet d'obtenir l'image 1 d'au moins une coupe plane 2 d'un objet 3. Cet appareil comprend une source 4 de rayons X, fournissant des impulsions de haute énergie, à une fréquence prédéterminée. Il comprend aussi un collimateur primaire 11a pour que ces impulsions traversent l'objet 3 au voisinage du plan définissant la coupe 2. Sur cette figure, on a représenté le faisceau impulsionnel I de façon très schématique. Dans un but de clarification, ce faisceau présente sur la figure une épaisseur très importante mais il est évident que son épaisseur est très faible, de sorte que les impulsions de rayons X traversent l'objet 3, au voisinage d'un plan qui peut être qualifié de plan de coupe.

Cet appareil comprend aussi des moyens 5, 6, 7 supportant l'objet et capables par exemple de le déplacer en translation et/ou en rotation. Ces moyens peuvent être constitués par une table 5 déplaçable en translation parallèlement à deux axes Y et Z perpendiculaires. Elle peut supporter une autre table 7, déplaçable en rotation autour d'un axe 8. Les moyens 6 permettent de commander les différents mouvements de translation et de rotation. Ces moyens connus dans l'état de la technique, ne sont pas décrits ici en détail. L'appareil comprend aussi une barrette 10 de détecteurs de mesure qui reçoivent les impulsions d'énergie atténuée ayant traversé l'objet, ainsi que la fente d'un collimateur secondaire 11b. Ces détecteurs fournissent respectivement sur des sorties 12, des impulsions électriques de détection, représentatives des impulsions d'énergie atténuée, provenant de l'objet 3. Enfin, l'appareil comporte des moyens de traitement et de visualisation qui permettent d'obtenir l'image de la coupe 2 de l'objet, à partir des signaux électriques impulsionnels fournis par les sorties 12 des détecteurs de la barrette de mesure 10.

Les moyens de traitement et de visualisation comprenent un ensemble 13 de circuits amplificateurs-intégrateurs ayant des entrées respectivement reliées aux sorties 12 des détecteurs de mesure. Ces circuits amplificateurs intégrateurs seront décrits plus loin en détail. Chaque amplificateur-intégrateur comporte une entrée pour commander les durées d'intégration de chaque signal impulsionnel fourni par le détecteur de mesure correspondant. L'ensemble des entrées de commande de durée de l'intégration est désigné par la référence 14b sur cette figure. Par ailleurs, chaque amplificateur intégrateur comprend une entrée pour commander la lecture du signal intégré, l'ensemble de ces entrées de commande portent la référence 14a.

Les moyens de traitement et de visualisation comportent aussi un calculateur 15 relié à une mémoire 16 et à des moyens de visualisation 17 permettant d'afficher la coupe 2 de l'objet 3. Ce calculateur présente des sorties 18a et 18b respectivement reliées aux entrées de commande 14a et 14b des circuits amplificateurs-intégrateurs 13. Le calculateur peut aussi être relié à une imprimante 19.

La mémoire 16 du calculateur 15 contient un programme de séquencement et de traitement pour commander chaque circuit amplificateur-intégrateur, comme on le verra plus loin en détail, pour que chaque impulsion électrique de détection fournie par le détecteur correspondant soit intégrée de la façon suivante : chaque impulsion est intégrée sur une première durée dite de mesure brute et, après un certain délai, sur au moins une deuxième durée. Chaque circuit amplificateur-intégrateur fournit donc sur une sortie, une première impulsion dite de mesure brute, et une deuxième impulsion dite de correction ; ces impulsions présentent respectivement les première et deuxième durées. L'amplitude de la première impulsion est représentative de l'énergie de l'impulsion atténuée reçue par le détecteur et provenant de l'objet. L'amplitude de la deuxième impulsion est représentative de la traînée de l'impulsion électrique de détection fournie par le détecteur et du courant de fuite ou d'obscurité de celui-ci et du rayonnement éventuel de l'objet. Une sortie 20 commune par exemple à l'ensemble des sorties des circuits intégrateurs 13, est reliée à une entrée 42 d'un convertisseur analogique-numérique 21 ; une sortie de ce convertisseur est reliée au calculateur 15 ; ce convertisseur fournit des valeurs numériques de mesures brutes et de corrections, respectivement représentatives des amplitudes des première et deuxième impulsions de mesure et de correction, pour chacun des détecteurs. Le calculateur traite ces valeurs numériques, pour founir les valeurs numériques effectives de mesures des énergies des impulsions atténuées reçues par les détecteurs. Ces valeurs numériques effectives sont enregistrées dans la mémoire 16 du calculateur 15. Les valeurs numériques effectives ainsi enregistrées pour tous les détecteurs sont utilisées par le calculateur pour commander l'affichage de l'image 1 de la coupe 2 sur l'écran des moyens de visualisation 17.

En pratique, pour accroître le rapidité du traitement des impulsions fournies par les détecteurs et pour simplifier les connexions, les circuits amplicateurs-intégrateurs sont de façon avantageuse regroupés en plusieurs ensembles ou sous-ensembles ; chaque sous-ensemble est relié à une entrée d'un convertisseur analogique-numérique dont la sortie est reliée au calculateur. Ces sous-ensembles regroupent par exemple respectivement dix circuits amplificateurs-intégrateurs.

Chaque convertisseur reçoit, en scrutant successivement par l'intermédiaire des entrées de commande 14a de lecture et de la sortie 20 commune, les signaux des circuits amplificateurs-intégrateurs auxquels il est relié, entre deux phases d'intégration.

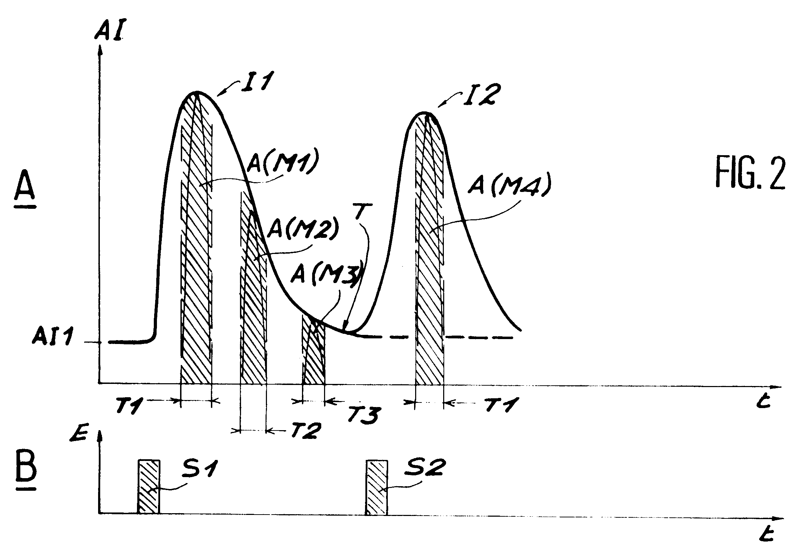

La figure 2 est un diagramme qui permet de mieux comprendre le séquencement et le traitement des signaux de détection fournis par les détecteurs. Sur cette figure, le diagramme A représente, en fonction du temps t, les variations d'amplitude AI de deux impulsions successives de détection I1, I2, fournies par la sortie de l'un des détecteurs 10.

Le diagramme B représente l'énergie E, en fonction du temps t, des rayonnements pulsés émis par la source 4. Ces variations d'énergie sont représentées ici en exemple, par deux impulsions successives S1, S2. Les impulsions I1, I2, de détection, à la sortie de l'un des détecteurs, sont synchrones avec les impulsions S1, S2. Chaque détecteur, en l'absence de réception d'une impulsion de rayonnement d'énergie atténuée, délivre un courant de fuite ou courant d'obscurité d'amplitude AI1. Les impulsions reçues par chaque détecteur présentant une énergie élevée, il en résulte que l'impulsion I1 de détection présente une traînée T qui perturbe l'impulsion de détection I2 dont l'amplitude de départ devrait être AI1.

Comme indiqué plus haut, le séquencement et le traitement consistent, pour chaque impulsion de détection telle que I1, à intégrer cette impulsion sur une première durée T1, dite de mesure brute, et à intégrer cette impulsion sur au moins une deuxième durée T2, dite de correction, dans l'intervalle de temps qui sépare cette impulsion de détection I1 de l'impulsion de détection I2 suivante. On peut aussi intégrer l'impulsion I1, sur une troisième durée T3 de correction, qui permet, comme on le verra plus loin en détail, d'effectuer une correction plus précise de la mesure de l'énergie reçue par le détecteur considéré. L'intégration de l'impulsion I1 sur la première durée T1 de mesure fournit une impulsion de mesure brute M1, dont l'amplitude est représentative de l'énergie de l'impulsion de rayonnement reçue par le détecteur, si l'on ne tient pas compte des perturbations dues à la traînée de l'impulsion précédente, au courant d'obscurité ou courant de fuite du détecteur et au rayonnement éventuel de l'objet. De la même manière, l'intégration de l'impulsion I1 sur la durée de correction T2, fournit une deuxième impulsion M2 dite de correction, dont l'amplitude est représentative de la traînée de l'impulsion I1, du courant de fuite ou d'obscurité du détecteur et du rayonnement éventuel de l'objet. La troisième impulsion M3 résultant de l'intégration sur une autre durée T3 de correction, présente elle aussi une amplitude A(M3) représentative de la traînée, du courant d'obscurité ou de fuite et du rayonnement éventuel de l'objet. Les impulsions de mesure et de correction M1, M2, M3, sont appliquées au convertisseur analogique numérique 21, par la sortie du circuit amplificateur-intégrateur. Le séquencement des périodes d'intégration est commandé par le calculateur 15. Les valeurs numériques correspondant aux amplitudes des impulsions M1, M2, M3, sont enregistrées dans la mémoire 16 du calculateur. Celui-ci corrige alors la valeur numérique représentative de l'amplitude M1 de l'impulsion de mesure brute de l'énergie reçue par le détecteur, en soustrayant à cette valeur numérique de mesure brute, une valeur numérique liée à l'amplitude de l'impulsion M2 de correction ou en soustrayant une valeur numérique liée aux amplitudes des impulsions M2 et M3 de correction. Comme les amplitudes des impulsions M2 et M3 sont représentatives de la traînée du signal I1 et du courant de fuite du détecteur correspondant et du rayonnement éventuel de l'objet, le calculateur enregistre alors en mémoire une valeur numérique effective représentant beaucoup plus précisément la valeur de l'énergie reçue par le détecteur.

On suppose dans cet exemple que la correction de mesure brute est effectuée, grâce aux valeurs numériques correspondant aux impulsions M2, M3 sur la valeur numérique de l'amplitude de l'impulsion M1. Il serait possible d'effectuer cette correction sur la valeur numérique de mesure brute de l'amplitude A(M4) de l'impulsion M4, représentant l'intégration sur la durée T1, de l'impulsion T2 suivante fournie par le détecteur. Il est donc possible de tenir compte de la traînée présente dans une impulsion de détection, soit sur cette impulsion, soit sur l'impulsion de détection suivante.

Selon un autre mode de réalisation, l'appareil de l'invention comporte une autre barrette de détecteurs 22, dite de compensation, identique à la barrette 10 de détecteurs de mesure ; cette barrette de détecteurs de compensation est située à proximité de la barrette de détecteurs de mesures. Ces barrettes de détecteurs seront décrites plus loin en détail ; la barrette de détecteurs de compensation comporte des moyens 23, tels qu'un blindage, la rendant opaque aux impulsions I d'énergie atténuée, provenant de l'objet 3.

Dans ce mode de réalisation, les effets du courant de fuite n'interviennent plus puisque ceux-ci sont compensés par la barrette de compensation et par une mesure différentielle des courants des détecteurs, comme on le verra plus loin en détail.

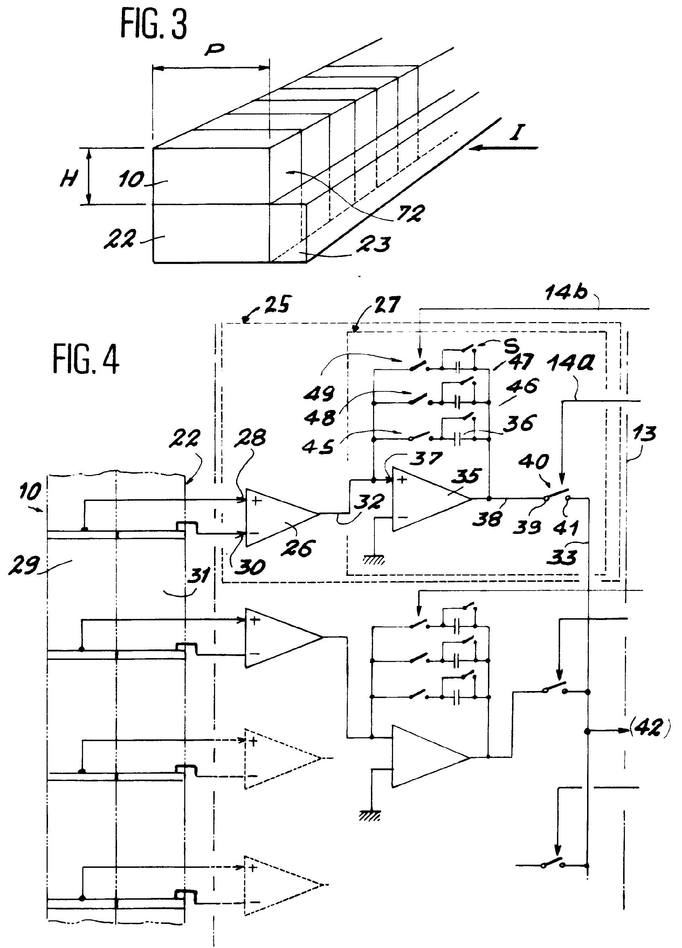

La barrette de détecteurs de mesure 10, la barrette de détecteurs de compensation 22 et le blindage 23, apparaissent mieux sur la figure 3. On comprend sur cette figure que le blindage 23 empêche les impulsions de rayonnement I de parvenir au détecteurs de compensation 22, alors que ces impulsions parviennent aux détecteurs de mesure 10. Le rôle de la barrette de détecteurs de compensation 22 sera décrit plus loin en détail.

La figure 4 représente schématiquement un premier mode de réalisation des circuits amplificateurs-intégrateurs 13 utilisés dans l'appareil de l'invention. Dans ce mode de réalisation, on suppose que l'appareil comprend une barrette 10 de détecteurs de mesure, recevant les impulsions de rayonnement provenant de l'objet, et une barrette 22 de détecteurs de compensation, munie d'un blindage (non représenté) la rendant insensible aux impulsions de rayonnement provenant de l'objet.

Dans ce mode de réalisation, chaque circuit amplificateur intégrateur tel que le circuit 25 de l'ensemble 13 comporte un amplificateur différentiateur 26 et un intégrateur 27. L'amplificateur différentiateur 26 présente un première entrée 28 reliée à une électrode du détecteur de mesure correspondant 29 de la barrette de mesure 10, et une deuxième entrée 30 reliée à une électrode du détecteur de compensation correspondant 31 de la barrette de compensation 22. Chaque amplificateur différentiateur, tel que l'amplificateur 26 fournit sur une sortie 32 des impulsions de détection amplifiées et compensées en fonction de la dérive en température et du courant d'obscurité du détecteur de mesure. Ces impulsions sont synchrones des impulsions reçues provenant de l'objet. Comme les détecteurs de mesure et de compensation sont identiques, et à la même température, ils subissent les mêmes dérives. Ces dérives sont donc compensées par l'amplificateur différentiateur 26. Le détecteur de compensation 31 présentant un blindage opaque aux impulsions de rayonnements reçus par le détecteur de mesure, le signal fourni par ce détecteur de compensation est également représentatif du courant d'obscurité du détecteur de mesure 29.

La sortie 32 de l'amplificateur différentiateur 26 est reliée à une entrée de l'intégrateur 27. Cet intégrateur comporte lesdites entrées 14a et 14b de commande, reliée à une sortie correspondante de command 18a et 18b du calculateur 15. Cet intégrateur fournit sur une sortie 33 lesdites impulsions de mesure et de correction obtenues par intégration sur au moins les première et deuxième durées mentionnées plus haut. Les autres circuits amplificateurs-intégrateurs reliés aux autres détecteurs de barrettes de mesure et de compensation, sont identiques au circuit amplificateur intégrateur qui vient d'être décrit.

Chaque circuit intégrateur 27 comprend un montage à amplificateur opérationnel. Ce montage comporte un amplificateur opérationnel 35 et au moins un condensateur d'intégration tel que 36. Cet amplificateur opérationnel reçoit sur son entrée 37, l'impulsion de détection amplifiée provenant de l'amplificateur différentiateur correspondant 26. Une autre entrée de l'amplificateur 35 est reliée à un potentiel de référence. Une sortie 38 du montage intégrateur à amplificateur opérationnel est reliée à une borne 39 d'un interrupteur 40 à commande d'ouverture et de fermeture. Cette commande est précisément réalisée par un signal fourni par la sortie de commande 18a de l'ordinateur 15. Ce signal est appliqué à l'entrée de commande 14a correspondant à l'interrupteur 40 successivement pour chaque circuit amplificateur-intégrateur de l'ensemble ou des sous-ensembles de ces circuits, entre deux intégrations. Une autre borne 41 de l'interrupteur 40 constitue la sortie 33 de l'amplificateur intégrateur 27. Cette sortie est une sortie commune aux circuits amplificateurs intégrateurs de l'ensemble ou d'un sous-ensemble de ces circuits ; elle est reliée à l'entrée 42 du convertisseur analogique numérique 21 correspondant.

De préférence, et selon les amplitudes des impulsions de détection à intégrer, chaque circuit amplificateur intégrateur comprend plusieurs condensateurs d'intégration 36, 46, 47 respectivement reliés entre la sortie et l'entrée de l'amplificateur opérationnel 35, au moyen d'interrupteurs 45, 48, 49 commandés par un signal fourni par la sortie de commande 18b de l'ordinateur 15 et appliqué à l'entrée de commande 14b. Les condensateurs 36, 46, 47 présentent des capacités de valeurs différentes. Le choix de l'un des condensateurs grâce à l'un des interrupteurs 45, 48, 49, permet de fixer de façon prédéterminée la quantité de charges maximales que chaque intégrateur peut prendre en compte. En effet, selon l'amplitude des impulsions de détection pour lesquelles des opérations d'intégration sont à effectuer, et selon l'échelle de conversion du convertisseur analogique numérique, il peut être utile de choisir de façon prédéterminée la quantité de charges maximales pour chaque intégrateur. Le choix des condensateurs est en outre lié aux durées d'intégration (T1, T2, T3) définies préalablement ; les durées d'intégration sont liées à la durée de fermeture des interrupteurs correspondants. Le choix des condensateurs et des durées d'intégration est effectué au cours d'une phase d'étalonnage préalable de l'appareil.

Après chaque intégration et de façon plus précise, après réception par le convertisseur correspondant, du signal de sortie du circuit amplificateur-intégrateur, il convient de mettre à zéro l'intégrateur correspondant en déchargeant l'un des condensateurs d'intégration 36, 46 ou 47, utilisé par exemple au moyen d'un transistor à effet de champ représenté sur la figure par un interrupteur S relié en parallèle avec le condensateur 47 considéré. Un interrupteur est relié en parallèle avec chaque condensateur d'intégration. On peut bien entendu avoir un seul interrupteur relié en parallèle à l'ensemble de ces condensateurs. La commande de la mise à zéro par le transistor est effectuée soit automatiquement de façon analogique par l'intermédiaire d'un circuit à retard lié à la commande de fermeture de l'interrupteur 40, soit directement par le calculateur à partir du programme de traitement.

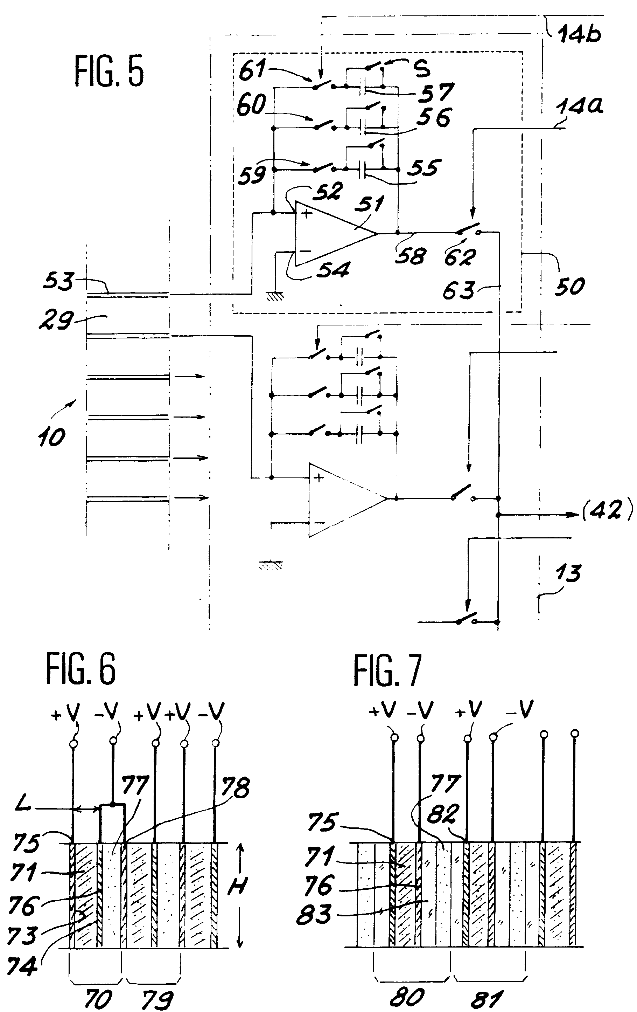

La figure 5 représente schématiquement un autre mode de réalisation de circuit amplificateur intégrateur utilisé dans l'appareil de l'invention. Seul l'un des circuits amplificateurs intégrateurs 50, appartenant à l'ensemble des circuits amplificateurs intégrateurs 13, sera décrit ici en détail. Dans ce mode de réalisation, on suppose que l'appareil ne comporte pas de barrette de détecteurs de compensation, mais seulement une barrette 10 de détecteurs de mesure. Dans ce cas, le circuit amplificateur intégrateur 50 comprend un montage intégrateur à amplificateur opérationnel 51, comparable au montage 27 de la figure 4.

Cet amplificateur opérationnel comporte une entrée 52 reliée à une électrode de sortie 53 d'un détecteur 29 correspondant. Cette électrode fournit des impulsions de détection. L'autre entrée 54 de l'amplificateur opérationnel 51 est reliée à un potentiel de référence. Comme dans le montage précédent, des condensateurs d'intégration 55, 56, 57 montés en parallèle entre l'entrée 52 de l'amplificateur opérationnel 51 et la sortie 58 de cet amplificateur, peuvent être respectivement sélectionnés par des interrupteurs 59, 60, 61 commandés par des signaux provenant de la sortie 18b du calculateur et appliqués à une entrée 14b. Le choix de l'un de ces condensateurs permet, de façon prédéterminée, de fixer la quantité de charges maximale du circuit intégrateur. Ce circuit intégrateur comporte aussi, comme dans le montage précédent, des moyens de mise à zéro représentés par des interrupteurs reliés respectivement aux condensateurs d'intégration et un interrupteur 62 dont l'ouverture ou la fermeture est commandée par des signaux provenant de la sortie 18a du calculateur et qui sont appliqués sur une entrée 14a. Cette entrée est l'une des entrées de commande 14a de l'ensemble ou d'un sous-ensemble de circuits amplificateurs-intégrateurs. Les interrupteurs 59, 60, 61 permettent comme précédemment, de fixer les différentes durées d'intégration décrites plus haut. Les autres circuits amplificateurs-intégrateurs sont identiques à celui qui vient d'être décrit. La sortie 63 de l'amplificateur intégrateur 50 forme une sortie commune à l'ensemble 20 ou à un sous-ensemble des sorties des circuits amplificateurs-intégrateurs. Elle est reliée à l'entrée 42 du convertisseur analogique numérique 21 correspondant.

Dans ce mode de réalisation, chaque circuit amplificateur-intégrateur fournit des impulsions de mesure et de correction, telles que décrites plus haut. Ces impulsions ne permettent pas ici de prendre en compte les dérives en température de la barrette 10 de détecteurs de mesure. Il est donc utile dans ce mode de réalisation d'associer la barrette 10 de détecteurs de mesure (la barrette de détecteur de compensation étant absente), à des moyens numériques 65 d'indication de température (figure 1). Ces moyens d'indication de température sont situés à proximité de la barrette 10 de détecteurs de mesure. Ces moyens numériques 65 fournissent sur une sortie un signal numérique représentatif de la valeur de la température à proximité de la barrette 10 de détecteurs de mesure. Cette sortie est reliée à une entrée du calculateur 15. Dans ce cas, la mémoire du calculateur comporte des tables d'étalonnage permettant de déterminer, pour chaque température, les dérives en courant des détecteurs de mesure. Le calculateur effectue alors, de manière connue, un calcul de correction des valeurs numériques de mesures effectives déjà enregistrées dans la mémoire et correspondant à la mesure de l'énergie des impulsions provenant de l'objet. La mesure de température permet d'améliorer la précision des valeurs effectives enregistrées.

Quel que soit le mode de réalisation choisi, l'appareil comprend de plus un détecteur de référence 67 (figure 1) permettant de mesurer l'énergie des impulsions provenant directement de la source 4. Ce détecteur de référence peut être, soit situé directement dans le faisceau d'impulsions I, c'est-à-dire dans la fente du collimateur 11a, soit situé comme représenté en 68, entre la source 4 et le collimateur 11a, en dehors du faisceau direct d'impulsions I émises vers l'objet 3. Ce détecteur de référence fournit sur une sortie des impulsions de détection d'énergie du références, représentatives de l'énergie de la source. Ces impulsions de référence sont appliquées à l'entrée 69 d'un convertisseur analogique numérique 70. Ce convertisseur fournit sur une sortie reliée au calculateur 15, une valeur numérique représentative de l'énergie de la source. Le détecteur 67 peut être constitué de manière connue et ne sera pas décrit ici en détail. Ce détecteur peut être aussi constitué comme l'un des détecteurs de mesure de la barrette 10 et peut éventuellement être associé à un des circuits amplificateur-intégrateur 64 de structure comparable à celle des amplificateurs-intégrateurs décrits plus haut. Le convertisseur analogique-numérique 70 reçoit une impulsion représentative de l'énergie de la source 4. Le détecteur de référence 67 est particulièrement important car la source étant pulsée et de haute énergie, les impulsions qu'elle fournit peuvent présenter des fluctuations d'énergie. Le calculateur enregistre les valeurs numériques de l'énergie des impulsions de la source, au cours du fonctionnement et corrige les valeurs numériques effectives enregistrées, en fonction des variation d'énergie de la source. Ces mesures d'énergie des impulsions de la source permettent d'améliorer la précision des valeurs effectives enregistrées.

La figure 6 représente un premier mode de réalisation d'une barrette de détecteurs utilisée dans l'appareil de l'invention. Ces détecteurs sont de type semi-conducteur à haute résistivité (supérieure à 10⁵Ω·cm). Ils peuvent utiliser par exemple, l'un des semi-conducteurs suivants : CdTe(Cl), HgI₂, AsGa, InP(Fe), Bi₁₂GeO₂₀, Bi₁₂SiO₂₀.

Chaque détecteur, tel que le détecteur 70 sur cette figure, comporte une pastille semi-conductrice 71 ayant la forme d'un parallélépipède allongé défini par sa largeur L, sa hauteur H, sa profondeur P (celle-ci étant supérieure ou égale à 1 cm) (voir figure 3). Sur la figure 6 seules apparaissent la hauteur H et la largeur L. Une face du parallélépipède 71 qui forme la pastille semi-conductrice, est située en regard de l'objet pour recevoir les impulsions de rayonnement provenant de cet objet (face 72 sur la figure 3). Deux autres faces parallèles 73, 74 de la pastille semi-conductrice, définies par la hauteur H et la profondeur P, portent respectivement deux électrodes 75 et 76 d'alimentation électrique (+V, -V). L'une des électrodes, telles que l'électrode 75 fournit les impulsions de détection mentionnées plus haut.

Chaque électrode est recouverte de façon avantageuse d'une couche 77 de blindage, opaque aux rayons diffusés par le détecteur. Cette couche de blindage permet de diminuer la diaphonie entre détecteurs voisins, autrement dit permet de limiter la diffusion de photons entre détecteurs voisins. Dans ce mode de réalisation, les électrodes 76 et 78 de deux détecteurs adjacents 70, 79, sont reliées à la borne commune d'alimentation -V. Dans le cas de cet exemple de réalisation, le blindage doit être électriquement isolant.

La figure 7 représente schématiquement un autre mode de réalisation des détecteurs de l'appareil de l'invention. Deux de ces détecteurs sont représentés en 80, 81 sur cette figure. Dans cet autre mode de réalisation, chaque détecteur comporte aussi une pastille semi-conductrice 71 de forme parallélépipédique, présentant sur deux faces parallèles, des électrodes 75, 76. Dans ce mode de réalisation, les électrodes adjacentes 76, 82 des deux détecteurs adjacents 80, 81, ne sont pas portées au même potentiel mais à des potentiels différents -V, +V. Par ailleurs, le blindage est par exemple métallique, il en résulte que chacun des détecteurs doit comporter, outre la couche de blindage 77, une couche électriquement isolante 83 située entre chaque électrode telle que 76 et la couche de blindage telle que 77.

La représentation donnée des détecteurs de mesure 10 et des détecteurs de compensation 22 correspond à des détecteurs 10 et 22 indépendants mais bien entendu ils peuvent de manière avantageuse être réalisés à partir d'une même pastille semi-conductrice présentant sur deux faces parallèles deux paires d'électrodes correspondant aux électrodes des détecteurs 10 et 22.

L'appareil qui vient d'être décrit permet bien d'atteindre les buts mentionnés plus haut : il permet d'obtenir une image satisfaisante d'une coupe d'un objet, grâce à une correction des mesures d'amplitude des impulsions de détection ; cette correction tient compte de la traînée de chaque signal impulsionnel fourni par chaque détecteur, du courant d'obscurité du rayonnement éventuel de l'objet, des dérives en températures et des fluctuations de l'énergie de la source impulsionnelle.

## Revendications

1. Appareil de tomographie a rayons X, pour obtenir l'image (1) d'au moins une coupe plane (2) d'un objet (3), comprenant une source (4) de rayons X fournissant des impulsions de haute énergie, à une fréquence prédéterminée, un collimateur primaire (5) pour que ces impultions traversent l'objet (3) au voisinage d'un plan de coupe, au moins une barrette (10) de détecteurs photoélectriques de mesure recevant les impulsions d'énergie atténuée ayant traversé l'objet et un collimateur secondaire (11), ces détecteurs fournissant respectivement sur des sorties (12) des impulsions électriques de détection, représentatives des impulsions d'énergie atténuée provenant de l'objet, des moyens de traitement et de visualisation reliés aux sorties des détecteurs de mesure pour fournir l'image (1) de ladite coupe (2) et des moyens (5, 6, 7) capables de déplacer en translation et/ou en rotation l'objet et/ou l'ensemble source, collimateurs primaire et secondaire, barrette de détecteurs, les moyens de traitement et de visualisation comprenant un ensemble de circuits amplificateurs-intégrateurs (13) ayant des entrées respectivement reliées aux sorties (12) des détecteurs de mesure (10), chaque amplificateur-intégrateur ayant une entrée (14b) pour commander la durée d'intégration, un calculateur (15) relié à une mémoire (16) et à des moyens de visualisation (17) et ayant des sorties de commande (18) respectivement reliées aux entrées (14a, 14b) de commande des circuits intégrateurs-amplificateurs (13), la mémoire (16) du calculateur (15) contenant un programme de séquencement et de traitement pour commander chaque circuit intégrateur-amplificateur pour que chaque impulsion de détection (I1) fournie par un détecteur correspondant, soit intégrée sur une première durée (T1) dite de mesure brute, et pour intégrer cette impulsion de détection sur au moins une deuxième durée (T2) dite de correction, dans l'intervalle de temps qui sépare cette impulsion de détection (I1) de l'impulsion de détection (I2) suivante, ce circuit intégrateur-amplificateur fournissant sur une sortie une première impulsion (M1) de mesure brute et une deuxième impulsion (M2) de correction ayant respectivement les première et deuxième durées (T1, T2), l'amplitude de la première impulsion (M1) étant représentative de l'énergie de l'impulsion atténuée provenant de l'objet et reçue par le détecteur, l'amplitude de la deuxième impulsion (M2) étant représentative de la traînée (T) de l'impulsion électrique de détection fournie par le détecteur et du rayonnement éventuel de l'objet et du courant de fuite du détecteur, les circuits amplificateurs-intégrateurs étant regroupés en au moins un ensemble, les circuits de chaque ensemble ayant une sortie commune (20) reliée à un convertisseur analogique-numérique (21) ayant une sortie reliée au calculateur (15) pour fournir des valeurs numériques de mesures brute et de correction, respectivement représentatives des amplitudes des première et deuxième impulsions (M1, M2) de mesure et de correction, le calculateur (15) traitant ces valeurs numériques pour chaque impulsion de détection (I1) pour fournir une valeur numérique effective de mesure de l'énergie de l'impulsion atténuée (I1) reçue par le détecteur, cette valeur numérique effective étant enregistrée dans la mémoire (16), les valeurs numériques effectives ainsi enregistrées pour tous les détecteurs étant utilisées par le calculateur (15) pour commander l'affichage de l'image (1) de ladite coupe (2) par les moyens de visualisation (17), chaque détecteur comportant une pastille semi-conductrice (71), chaque pastille ayant la forme d'un parallélépipède allongé défini par sa largeur (L), sa hauteur (H) et sa profondeur (P), une face (72) de ce parallélépipède définie par la largeur et la hauteur étant située en regard de l'objet, deux autres faces parallèles de ce parallélépipède définies par la hauteur et la profondeur portant respectivement deux électrodes (73, 74) d'alimentation électrique, l'une de ces électrodes (73) fournissant lesdites impulsions de détection.

2. Appareil selon la revendication 1, caractérisé en ce qu'il comprend en outre une barrette (22) de détecteurs de compensation, identique à la barrette (10) de détecteurs de mesure et située à proximité de cette barrette de détecteurs de mesure, cette barrette (22) de détecteurs de compensation comportant des moyens (23) pour la rendre opaque aux impulsions d'énergie atténuées provenant de l'objet, chaque circuit amplificateur-intégrateur (25) comportant un amplificateur différentiateur (26) ayant une première entrée reliée au détecteur de mesure (29) correspondant, et une deuxième entrée reliée au détecteur de compensation (31) correspondant, chaque amplificateur différentiateur (26) fournissant sur une sortie une impulsion de détection amplifiée et compensée en fonction de la dérive en température et du courant d'obscurité du détecteur de mesure (29) correspondant, la sortie de cet amplificateur différentiateur étant reliée à une entrée d'un intégrateur (27) ayant l'une desdites entrées de commande (14b) de durée d'intégration et fournissant sur une sortie (33) lesdites impulsions (M1, M2) de mesure et de correction.

3. Appareil selon la revendication 1, caractérisé en ce que chaque circuit amplificateur-intégrateur (50) comprend un montage intégrateur à amplificateur opérationnel (51), une entrée de cet amplificateur opérationnel recevant l'impulsion de détection provenant du détecteur (29) correspondant, une entrée de commande de la durée d'intégration du montage intégrateur étant reliée à une sortie de commande correspondante du calculateur (15), une sortie (58) de cet amplificateur opérationnel étant reliée à une borne d'un interrupteur (62) ayant une entrée (14a) de commando d'ouverture ou de fermeture reliée à une sortie de commande (18) correspondante du calculateur (15), une autre borne de cet interrupteur étant reliée à l'entrée (42) du convertisseur analogique-numérique (21) correspondant.

4. Appareil selon la revendication 2, caractérisé en ce que chaque intégrateur (27) comprend un montage intégrateur à amplificateur opérationnel (35), une entrée de cet amplificateur opérationnel recevant l'impulsion amplifiée de détection provenant de l'amplificateur différentiateur correspondant (26), une entrée de commande de la durée d'intégration du montage intégrateur étant reliée a une sortie de commande correspondante du calculateur (15), une sortie (38) du montage à amplificateur opérationnel étant reliée à une borne d'un interrupteur (40) ayant une entrée (14) de commande d'ouverture ou de fermeture reliée à une sortie de commande correspondante (18) du calculateur (15), une autre borne de cet interrupteur étant reliée à l'entrée (42) du convertisseur analogique-numérique (21) correspondant.

5. Appareil selon la revendication 3, caractérisé par le fait qu'il comporte des moyens numériques (65) d'indication de température situés à proximité de la barrette (10) de détecteurs de mesure, une sortie de ces moyens d'indication fournissant un signal numérique d'indication de la valeur de la température à proximité de la barrette (10) de détecteurs de mesure, cette sortie étant reliée à une entrée du calculateur (15) pour que celui-ci effectue un traitement de correction des valeurs numériques de mesures effectives enregistrées, pour corriger ces valeurs numériques selon la dérive en température des détecteurs de mesure.

6. Appareil selon l'une quelconque des revendications 4 et 5, caractérisé en ce qu'il comporte un détecteur de référence (67) recevant les impulsions de la source (4) et fournissant sur une sortie des impulsions de détection de référence représentatives de l'énergie de la source, un convertisseur analogique-numérique (70) ayant une entrée reliée à la sortie du détecteur de référence et fournissant sur une sortie, une valeur numérique représentative de l'énergie de la source (4), cette sortie étant reliée à une entrée du calculateur (15) pour que celui-ci effectue un traitement de correction des valeurs numériques effectives enregistrées, pour corriger ces valeurs selon les variations d'énergie de la source (4).

7. Appareil selon la revendication 6, caractérisé en ce que chaque électrode est recouverte d'une couche de blindage (77) opaque aux rayons reçus par le détecteur et évitant la diffusion dans chaque détecteur voisin.

8. Appareil selon la revendication 7, caractérisé en ce qu'une couche (83) électriquement isolante est intercalée entre chaque électrode (76) et la couche de blindage (77).

## Patentansprüche

1. Vorrichtung für Röntgenstrahltomographie, um das Bild (1) von mindestens einer Schnittebene (2) eines Objekts (3) zu erhalten, die eine Röntgenstrahlungsquelle (4), welche hochenergetische Impulse bei einer vorher bestimmten Frequenz liefert, einen ersten Kollimator (5), damit diese Impulse das Objekt (3) in der Nähe der Schnittebene durchlaufen, mindestens einen Steg (10) photoelektrischer Meßdetektoren, welche die gedämpften Energieimpulse, die das Objekt durchlaufen haben, empfangen, und einen zweiten Kollimator (11) umfaßt, wobei diese Detektoren entsprechend an den Ausgängen (12) elektrische Detektionsimpulse, repräsentativ für die gedämpften Energieimpulsen, die von dem Objekt stammen, liefern, Geräte zur Verarbeitung und Anzeige sind mit den Ausgängen der Meßdetektoren verbunden, um das Bild (1) des besagten Schnitts (2) zu liefern und Geräte (5,6,7), die fähig sind, das Objekt und/oder die Quelleneinheit in der Translation und/oder in der Rotation zu verschieben, erste und zweite Kollimatoren, Detektorstege, die Geräte zur Verarbeitung und Anzeige, welche eine Schaltungseinheit Verstärker-Integrator (13) umfassen, die Eingänge hat, die entsprechend mit den Ausgängen (12) der Meßdetektoren (10) verbunden sind, wobei jeder Verstärker-Integrator einen Eingang (14b) hat, um die Integrationsdauer zu steuern, ein Rechner (15), der mit einem Speicher (16) und Anzeigegeräten (17) verbunden ist und welcher Steuerausgänge (18) hat, die entsprechend mit den Eingängen (14a, 14b) zur Steuerung der Integrator-Verstärker-Schaltungen (13) verbunden sind, der Speicher (16) des Rechners (15) enthält ein Programm zum Ablauf und zur Verarbeitung, um jede Integrator-Verstärker-Schaltung zu steuern, damit jeder Detektorimpuls (I1), der von dem entsprechenden Detektor geliefert wird, sowohl in einer ersten Dauer (T1), dem sogenannten Rauschmessen, integriert wird und um diesen Detektorimpuls in mindestens einer zweiten Dauer (T2), der sogenannten Korrektur, in einem Zeitintervall zu integrieren, das diesen Detektorimpuls (I1) vom folgenden Detektorimpuls (I2) trennt, diese Integrator-Verstärker-Schaltung ,welche an einem Ausgang einen ersten Impuls (M1) der Rauschmessung und einen zweiten Impuls (M2) der Korrektur liefert, hat entsprechend die ersten und zweiten Dauern (T1, T2), die Amplitude des ersten Impulses (M1) ist repräsentativ für die Energie des gedämpften Impulses, der von dem Objekt stammt und von dem Detektor empfangen wird, die Amplitude des zweiten Impulses (M2) ist repräsentativ für den Widerstand (T) des elektrischen Detektionsimpulses, der von dem Detektor und von der eventuellen Strahlung des Objekts und von dem Spalt des Detektors geliefert wird, die Verstärker-Integrator-Schaltungen werden in mindestens einer Einheit zusammengefaßt, wobei die Schaltungen jeder Einheit einen gemeinsamen Ausgang (20) haben, der mit einem analog-digital-Konverter (21) verbunden ist, der einen Ausgang hat, welcher mit dem Rechner (15) verbunden ist, um digitale Werte der Rauschmesung und der Korrektur zu liefern, entsprechend repräsentativ für die Amplituden der ersten und zweiten Impulse (M1, M2) der Messung und der Korrektur, der Rechner (15) verarbeitet diese digitalen Werte für jeden Detektorimpuls (I1), um einen effektiven digitalen Wert der Messung der Energie der gedämpften Impulse (I1), die von dem Detektor empfangen wurden, zu liefern, diese effektiven digitalen Werte werden in dem Speicher (16) gespeichert, die somit für alle Detektoren gespeicherten effektiven digitalen Werte werden von dem Rechner (15) verwendet, um die Bildanzeige (1) des besagten Schnitts (2) durch Anzeigegeräte (17) zu steuern, wobei jeder Detektor ein halbleitendes Substrat umfaßt, jedes Substrat (71) hat die Form eines gestreckten Parallelflachs hat, das durch seine Breite (L), seine Höhe (H) und seine Tiefe (P) definiert ist, eine Fläche (72) dieses Parallelflachs, die durch die Breite und die Höhe definiert ist, wird gegenüber dem Objekt angeordnet, zwei andere parallele Flächen dieses Parallelflachs, die durch die Breite und die Tiefe definiert werden, tragen entsprechend zwei Elektroden (73, 74) zur Stromversorgung, wobei eine dieser Elektroden (73) die besagen Detektorimpulse liefert.

2. Vorrichtung gemäß Anspruch 1, dadurch gekennzeichnet, daß sie außerdem einen Steg (22) von Kompensationsdetektoren umfaßt, der mit dem Steg (10) der Meßdetektoren identisch ist und in der Nähe dieses Stegs der Meßdetektoren angeordnet ist, dieser Steg (22) der Kompensationsdetektoren umfaßt Geräte (23), um sie für die gedämpften Energieimpulse, die von dem Objekt stammen, undurchlässig zu machen, jede Verstärker-Integrator-Schaltung (25), welche einen Differentialverstärker (26) umfaßt, hat einen ersten Eingang, der mit dem entsprechenden Meßdetektor (29) verbunden ist, und einen zweiten Eingang, der mit dem entsprechenden Kompensationsdetektor (31) verbunden ist, jeder Differentialverstärker (26), welcher an einem Ausgang einen verstärkten Detektorimpuls liefert und in Abhängigkeit von der Temperaturabweichung und des Dunkelstroms des entsprechenden Meßdetektors (29) kompensiert ist, der Ausgang dieses Differentialverstärkers ist mit einem Eingang eines Integrators (27) verbunden, der einen der besagten Steuereingänge (14b) der Integrationsdauer hat und an einem Ausgang (33) die besagen Impulse (M1, M2) der Messung und der Korrektur liefert.

3. Vorrichtung gemäß Anspruch 1, dadurch gekennzeichnet, daß jede Verstärker-Integrator-Schaltung (50) einen integrierenden Einbau mit im Einsatz befindlichem Verstärker (51) umfaßt, wobei ein Eingang dieses im Einsatz befindlichen Verstärkers die Detektorimpulse empfängt, die von dem entsprechenden Detektor (29) stammen, ein Steuereingang die Integrationsdauer des integrierenden Einbaus ist mit einem entsprechenden Steuerausgang des Rechners (15) verbunden, ein Ausgang (58) dieses im Einsatz befindlichen Verstärkers ist mit einer Anschlußklemme eines Unterbrechers (62) verbunden, die einen Eingang (14a) zur Steuerung des Öffnens oder Schließens, verbunden mit einem entsprechenden Steuerausgang (18) des Rechners (15) hat, eine andere Anschlußlemme dieses Unterbrechers ist mit dem Eingang (42) des entsprechenden analog-digital Umwandlers (21) verbunden.

4. Vorrichtung gemäß Anspruch 2, dadurch gekennzeichnet, daß jeder Integrator (27) einen integrierenden Einbau mit im Einsatz befindlichen Verstärker (35) umfaßt, wobei ein Eingang dieses im Einsatz befindlichen Verstärkers den verstärkten Impuls des Detektors empfängt, der von dem entsprechenden Differentialverstärker (26) stammt, ein Eingang zur Steuerung der Integrationszeit des integrierenden Einbaus wird mit einem entsprechenden Steuerausgang des Rechners (15) verbunden, ein Ausgang (38) des Einbaus mit im Einsatz befindlichen Verstärker wird mit einer Anschlußklemme eines Unterbrechers (40) verbunden, welcher einen Eingang (14) zur Steuerung des Öffnens oder Schließens hat, der mit einem entsprechenden Steuerausgang (18) des Rechners (15) verbunden ist, eine andere Anschlußklemme dieses Unterbrechers wird mit dem Eingang (42) des entsprechenden Analog-digital-Umwandlers (21) verbunden.

5. Vorrichtung gemäß Anspruch 3, dadurch gekennzeichnet, daß sie digitale Geräte (65) zur Temperaturanzeige umfaßt, die in der Nähe der Stege (10) der Meßdetektoren angeordnet sind, ein Ausgang dieser Anzeigegeräte, welcher ein digitales Anzeigesignal des Temperaturwerts in der Nähe des Steges (10) der Meßdetektoren liefert, dieser Ausgang wird mit einem Eingang des Rechners (15) verbunden, damit dieser eine Verarbeitung zur Korrektur der digitalen Werte der effektiven gespeicherten Messungen vornimmt, um die digitalen Werte gemäß der Temperaturabweichung der Meßdetektoren zu korrigieren.

6. Vorrichtung gemäß einem der Ansprüche 4 und 5, dadurch gekennzeichnet, daß sie einen Bezugsdetektor (67) umfaßt, welcher die Impulse der Quelle (4) empfängt und an einen Ausgang Bezugsimpulse der Detektion liefert, die für die Quellenenergie repräsentativ sind, ein analog-digital Umwandler (70), welcher einen Eingang hat, der mit dem Ausgang des Bezugsdetektors verbunden ist und an einem Ausgang einen digitalen Wert liefert, der für die Energie der Quelle (4) repräsentativ ist, dieser Ausgang wird mit einem Eingang des Rechners (15) verbunden, damit dieser eine Korrekturverarbeitung der effektiven gespeicherten digitalen Werte vornimmt, um diese Werte gemäß den Energieveränderungen der Quelle (4) zu korrigieren.

7. Vorrichtung gemäß Anspruch 6, dadurch gekennzeichnet, daß jede Elektrode mit einer Schicht zur Abschirmung (77) bedeckt ist, die für die von dem Detektor erhaltenen Strahlen undurchlässig ist und die Diffusion in jeden Nachbardetektor verhindert.

8. Vorrichtung gemäß Anspruch 7, dadurch gekennzeichnet, daß eine elektrisch isolierende Schicht (83) zwischen jede Elektrode (76) und die Schicht zur Abschirmung (77) dazwischen gelagert ist.

## Claims

1. X-ray tomography device for obtaining the image (1) of at least one plane (2) cutting of an object (3) and comprising an X-ray source (4) supplying high-energy pulses at a predetermined frequency, a primary collimator (5) so that these pulses traverse the object (3) close to a cutting plane, at least one bar (10) of photoelectric measuring detectors receiving the attenuated energy pulses having traversed the object and a secondary collimator (11), these detectors respectively supplying on outputs (12) electric detection pulses representative of the attenuated energy pulses originating from the object, display and processing means connected to the outputs of the measuring detectors so as to supply the image (1) of said cutting (2) and means (5,6,7) able to translation and/or rotation move the object and/or the source unit, primary and secondary collimators and the bar of detectors, the display and processing means including a set of integrator-amplifier circuits (13) having inputs respectively connected to the outputs (12) of the measuring detectors (10), each integrator-amplifier having one input (14b) so as to control the integration period, a computer (15) connected to a memory (16) and to display means (17) and having control outputs (18) respectively connected to the control inputs (14a,14b) of the integrator-amplifier circuits (13), the memory (16) of the computer (15) containing a sequencing and processing program so as to control each integrator-amplifier circuit so that each detection pulse (I1) supplied by a corresponding detector is integrated on a first rough measurement period (T1) and so as to integrate this detection pulse on at least one second correction period (T2) within the time interval separating this detection pulse (I1) from the next detection pulse (I2), this integrator-amplifier circuit supplying on one output a first rough measurement pulse (M1) and a second correction pulse (M2) having respectively the first and second periods (T1,T2), the amplitude of the first pulse being representative of the energy of the attenuated pulse originating from the object and received by the detector, the amplitude of the second pulse (T2) being representative of the drag (T) of the electric detection pulse supplied by the detector and any possible radiation from the object, and the stray current of the detector, the integrator-amplifier circuits being regrouped into at least one unit, the circuits of each unit having one common output (20) connected to an analog to digital converter (21) having one output connected to the computer (15) so as to supply numerical rough measurement and correction values respectively representative of the amplitudes of the first and second correction and measurement pulses (M1,M2), the computer (15) processing these numerical values for each detection pulse (I1), so as to supply one actual numerical value measuring the energy of the attenuated pulse received by the detector, this actual numerical value being recorded in the memory (16), the actual numerical values thus recorded for all the detectors being used by the computer (15) so as to control display of the image (1) of said cutting (2) by the display means (17), each detector including a semiconductor pellet (71), each pellet being shaped like an elongated parallelepiped defined by its width (L), its height (H) and its depth (P), one face (72) of said parallelepiped defined by the width and height being positioned facing the object, two other parallel faces of said parallelepiped defined by the height and the depth respectively carrying the two electric power supply electrodes (73,74), one of the said electrodes (73) supplying the said detection pulses.

2. Device according to claim 1, characterized in that it includes in addition a bar (22) of compensation detectors identical to the bar (10) of measuring detectors and situated close to this bar of measuring detectors, this bar (22) of compensation detectors comprising means (23) to render it opaque to the attenuated energy pulses derived from the object, each integrator-amplifier circuit (25) comprising a differential amplifier (26) having one input connected to the corresponding measuring detector (29), and a second input connected to the corresponding compensation detector (31), each differential amplifier (26) supplying on one output a detection pulse amplified and compensated according to the temperature drift and the dark current of the corresponding measuring detector (29), the output of this differential amplifier being connected to an integrator (27), one of said control inputs (14b) having an integration period and supplying on one output (33) said measuring and correction pulses (M1,M2).

3. Device according to claim 1, characterized in that each integrator-amplifier circuit (50) includes an operational amplifier (51) integrator connection, one input of this operational amplifier receiving the detection pulse originating from the corresponding detector (29), one input for controlling the integration period of the integrator connection being connected to one corresponding output of the computer (15), one output (58) of this operational amplifier being connected to a terminal of a circuit breaker (62) having one closing or opening control input (14a) connected to one corresponding control output (18) of the computer (15), another terminal of this circuit breaker being connected to the input (42) of the corresponding analog to digital converter (21).

4. Device according to claim 2, characterized in that each integrator (27) includes an operational amplifier (35) integrator connection, one input of this operational amplifier receiving the amplified detection pulse derived from the corresponding differential amplifier (26), one input for controlling the integration period of the integrator connection being connected to a corresponding control output of the computer (15), one output (38) of the operational amplifier connection being connected to a terminal of a circuit breaker (40) having one opening or closing control input (14) connected to one corresponding output (18) of the computer (15), another terminal of this circuit breaker being connected to the input (42) of the corresponding analog to digital converter (21).

5. Device according to claim 3, characterized in that it comprises numerical temperature indication means (65) situated close to the bar (10) of measuring detectors, one output of these indication means supplying one digital signal indicating the value of the temperature close to the bar (10) of measuring detectors, this output being connected to one input of the computer (15) so that the latter can carry out a correction processing of the actual measurement values recorded so as to correct these numerical values according to the temperature drift of the measuring detectors.

6. Device according to either of the claims 4 and 5, characterized in that it comprises a reference detector (67) receiving the pulses of the source (4) and supplying on one output reference detection pulses representative of the energy of the source, one analog to digital converter (70) having one input connected to the output of the reference detector and supplying on one output a numerical value representative of the energy of the source (4), this output being connected to one input of the computer (15) so that the latter can carry out a correction processing of the actual recorded numerical values in order to correct these values according to the energy variations of the source (4).

7. Device according to claim 6, characterized in that each electrode is covered with a screening layer (77) opaque to the rays received by the detector and avoiding diffusion into each neighbouring detector.

8. Device according to claim 7, characterized in that an electrically insulating layer (83) is inserted between each electrode (76) and the screening layer (77).
